# EUROPEAN PATENT SPECIFICATION

(11) **EP 2 089 072 B1**
(45) Date of publication and mention of the grant of the patent: **04.06.2014**
(21) Application number: 07837562.3
(22) Date of filing: 30.08.2007
(51) Int. Cl.: A61L 27/22, A61L 27/58, A61L 31/04, A61L 31/14

(54) **MEDICAL DEVICES HAVING A COATING FOR PROMOTING ENDOTHELIAL CELL ADHESION**
MEDIZINPRODUKTE MIT EINER BESCHICHTUNG ZUR FÖRDERUNG DER ADHÄSION VON ENDOTHELZELLEN
DISPOSITIFS MÉDICAUX AYANT UN REVÊTEMENT POUR FAVORISER L'ADHÉSION DES CELLULES ENDOTHÉLIALES

(30) Priority: 06.09.2006 US 842384 P
(43) Date of publication of application: 19.08.2009
(60) Divisional application: 11191939.5
(73) Proprietor: Boston Scientific Limited, Christ Church (BB)
(72) Inventor: BENCO, John, Holliston, MA 01746 (US); BODEN, Mark, Harris, RI 02830 (US); BRITO, Shaina, Winchester, MA 01890 (US); NAIMARK, Wendy, Cambridge, MA 02138 (US); PHAM, Lan, Nashua, NH (US)
(74) Representative: Vossius & Partner
(86) International application number: PCT/US2007/019109
(87) International publication number: WO 2008/030388

(56) References cited:
- EP-A- 1 632 258
- US-A1- 2005 147 647
- US-A1- 2005 187 146
- LESTINI B J ET AL: "Surface modification of liposomes for selective cell targeting in cardiovascular drug delivery" JOURNAL OF CONTROLLED RELEASE, ELSEVIER, AMSTERDAM, NL, vol. 78, no. 1-3, 17 January 2002 (2002-01-17), pages 235-247, XP004329821 ISSN: 0168-3659
- LIN H-B ET AL: "SYNTHESIS, SURFACE, AND CELL-ADHESION PROPERTIES OF POLYURETHANES CONTAINING COVALENTLY GRAFTED RGD-PEPTIDES" JOURNAL OF BIOMEDICAL MATERIALS RESEARCH, WILEY, NEW YORK, NY, US, vol. 28, no. 3, 1 March 1994 (1994-03-01), pages 329-342, XP001098048 ISSN: 0021-9304 cited in the application
- BAKER J.E.; NIKOLAYCHIK V.; ZULICH A.; KOMOROWSKI R.; KIPSHIDZE N.: "Fibrin Coated Stents as a Depot to Deliver RGD Peptide Inhibit Vascular Reaction in Atherosclerotic Rabbit Model" JOURNAL OF THE AMERICAN COLLEGE OF CARDIOLOGY, vol. 27, no. 2, February 1996 (1996-02), pages 197-197, XP002526567

## Description

The present invention relates to implantable or insertable medical devices having bioactive coatings thereon.

A problem associated with the use of vascular stents is reocclusion (restenosis) of the blood vessel after stent implantation. An important factor contributing to restenosis is the injury to or loss of the natural protective lining of endothelial cells on the inner surface of the artery as a result of stent implantation. This loss of the endothelial cell lining denudes the arterial wall, making it vulnerable to thrombosis, infection, scarring, or abnormal tissue growth. Thus, reestablishing a layer of endothelial cells (re-endothelialization) in the stented artery is thought to be important in improving the long-term biocompatibility of the stent. To promote effective endothelialization, however, endothelial cells must migrate from adjacent areas of the artery and adhere onto the surface of the stent.

It is known that certain proteins in the extracellular matrix, such as laminin, fibronectin, and collagen, are responsible for promoting endothelial cell adhesion. Additionally, various bioactive peptide sequences derived from these proteins, such as RGD and YIGSR, have been discovered to provide good substrates for endothelial cell adhesion.

Therefore, one approach to promoting re-endothelialization is by providing a surface coated with such bioactive peptides, such as the peptide-coated stent described in U.S. Patent Publication No. 2006/0052862 (Kanamaru et al.). Some have suggested that the peptides be incorporated into the backbone of polymers such as polyurethane, as described in U.S. Patent. Publication No. 2006/0067909 (West et al.), which is incorporated by reference herein; or be grafted onto polymers, as described in Lin et al., Synthesis, Surface, and Cell-Adhesion Properties of Polyurethanes Containing Covalently Grafted RGD-Peptides, J. Biomed. Materials Res. 28(3):329-42 (1994).

One of the problems associated with the use of such bioactive peptides *in vivo* is biofouling of the peptides caused by the binding of plasma proteins or platelets onto the peptides. This biofouling defeats the ability of the peptides to bind to the target endothelial cells. One suggested approach to preventing biofouling is to incorporate the peptides into a hydrophilic polymer and grafting polyethylene glycol (PEG) onto the polymer. However, this method for protecting the peptides against biofouling has certain disadvantages. Thus, there is a need for an alternate method of preventing the biofouling of bioactive peptides. There is also a need for alternate methods of coating medical devices with bioactive peptides.

US 2005/0187146 A1 discloses a biomaterial which comprises at least one biocompatible polymer in combination with at least one bioactive polymer comprised of at least one peptide and/or protein and at least one polysaccharide and/or proteoglycan.

The present invention provides a medical device having a coating over at least a portion of the medical device, wherein the coating comprises:
(a) one or more cell adhesion polypeptides; and
(b) a biodegradable barrier comprising a biodegradable polymer at least partially disposed over or surrounding the one or more cell adhesion polypeptides, wherein the biodegradable barrier substantially degrades within 7 days after implantation of the medical device in a patient.

In one embodiment, the cell adhesion polypeptides are peptides derived from a binding domain of a cell adhesion protein of the extracellular matrix.

In one embodiment, the cell adhesion protein is selected from the group consisting of fibronectin, vitronectin, laminin, elastin, fibrinogen, collagen type I, collagen type II, and collagen type V.

In one embodiment, the peptides comprise an amino acid sequence selected from the group consisting of arginine-glycine-aspartate (RGD) and tyrosine-isoleucine-glycine-serine-arginine (YIGSR).

In one embodiment, the cell adhesion polypeptides form a monolayer on the surface of the medical device.

In one embodiment, the biodegradable barrier comprises biodegradable micelles, and wherein the polypeptides are encapsulated within the micelles.

In one embodiment, the biodegradable barrier substantially degrades within 4 days after implantation of the medical device in a patient.

In one embodiment, the cell adhesion polypeptides are grafted onto a polybisphosphonate.

In one embodiment, the cell adhesion polypeptides are linked to adhesive polypeptide segments.
FIG. 1 shows a fragment of a polybisphosphonate with a cell adhesion peptide grafted thereon.
FIG. 2 is a schematic cross-section representation of a medical device according to the present invention with cell adhesion polypeptides carried on antibodies.
FIG. 3 is a schematic cross-section representation of a medical device according to the present invention with a coating of modified cell adhesion polypeptides.
FIG. 4 shows a fragment of a polybisphosphonate with a cell adhesion polypeptide-displaying bacteriophage grafted thereon.

### DETAILED DESCRIPTION

The present invention provides an implantable or insertable medical device having a coating of cell adhesion polypeptides to provide a substrate for the adhesion of endothelial cells onto the medical device. As used herein, the term "cell adhesion polypeptides" refers to compounds having at least two amino acids per molecule which are capable of binding endothelial cells via cell surface molecules, such as integrin, on endothelial cells. The cell adhesion polypeptides may be any of the proteins of the extracellular matrix which are known to play a role in cell adhesion, including fibronectin, vitronectin, laminin, elastin, fibrinogen, collagen types I, II, and V, as described in Boateng et al., RGD and YIGSR Synthetic Peptides Facilitate Cellular Adhesion Identical to That of Laminin and Fibronectin But Alter the Physiology of Neonatal Cardiac Myocytes, Am. J. Physiol. - Cell Physiol. 288:30-38 (2005). Additionally, the polypeptides may be any peptide derived from any of the aforementioned proteins, including fragments or sequences containing the binding domains. Such peptides include those having integrin-binding motifs, such as the RGD (arginine-glycine-aspartate) motif, the YIGSR (tyrosine-isoleucine-glycine-serine-arginine) motif, and related peptides that are functional equivalents. The peptides may also be any of the peptides described in U.S. Patent Publication No. 20060067909 (West et al.).

The cell adhesion polypeptides may be disposed on or within various types of surfaces on the medical device. In certain embodiments, the surface is the bare, uncoated surface of the medical device. The bare surface of the medical device may be smooth or porous, such as the porous stent surface described in U S Patent Publication No. 2005/0266040 (Gerberding).

Where the surface is porous, the cell adhesion polypeptides may be deposited within the pores of the porous surface In other embodiments, the surface of the medical device may be the surface of a coating on the medical device, such as a polymer coating In any of the embodiments of the present invention, the polypeptides may be bonded to the surface of the medical device by any type of chemical or physical bonding means, including covalent, polar, ionic, coordinate, metallic, electrostatic, or intermolecular dipolar (including Van der Waals) bonds.

The cell adhesion polypeptides can be applied onto the surface of the medical device in various ways, including the use of coating methods that are known in the art For example, the polypeptides may be sprayed onto the medical device by a conventional electrostatic spraying process, resulting in charged peptide-containing droplets being deposited onto the medical device. As the coating fluid dries, the polypeptides remain adhered to the medical device by inter-molecular bonding with the side-chain groups on the polypeptides. The deposited polypeptides may form a monolayer on the surface of the medical device, such as a Langmuir monolayer or a self assembling monolayer as described in Van Alsten, Self-Assembled Monolayers on Engineering Metals Structure, Derivation, and Utility, Langmuir 15 7605-14 (1999).

In certain embodiments, the cell adhesion polypeptides are incorporated into a polymer, which is then deposited onto a stent Within certain embodiments, the polypeptides may be incorporated into the backbone of a polymer chain For example, a polymer can be created containing YIGSR in the backbone of polyurethane as described in Jun et al., Development of a YIGSR-Peptide-Modified Polyurethaneurea to Enhance Endolhelialization, J. Biomaterials Sci , Polymer Ed. 15 (1) 73-94 (2004). One of skill in the art could incorporate other cell adhesion polypeptides into the backbone of polyurethane or other polymers.

Within certain embodiments, the cell adhesion polypeptides may be grafted onto a polymer, which is then deposited onto the medical device The polypeptides may be grafted onto a polymer using various methods known in the art. In one method, polymers having side branches containing reactive functional groups such as epoxide, halide, amine, alcohol, sulfonate, azido, anhydride, or carboxylic acid moieties can be covalently linked to the amine terminus of the polypeptides via the reactive side branches using conventional coupling techniques such as carbodiimide reactions. For example, RGD-containing peptides have been grafted onto the backbone of polyurethane, as described in Lin et al., Synthesis, Surface, and Cell-Adhesion Properties of Polyurethanes Containing Covalently Grafted RGD-Peptides, J. Biomedical Materials Res. 28(3):329-42 (1994). In another example, RGD-containing peptides have been grafted onto the side branches of polyethylene glycol based polymers, as described in Hansson et al., Whole Blood Coagulation on Protein Absorption-Resistant PEG and Peptide Functionalised PEG-Coated Titanium Surfaces, Biomaterials 26:861-872 (2005). One of ordinary skill in the art will also appreciate that polypeptides can be coupled to polymers via the carboxy-terminus of the polypeptides. For example, polymers with amine or hydroxyl side groups can be coupled to the carboxy-terminus of polypeptides by carbodiimide or condensation reactions to create an amide or ester linkage.

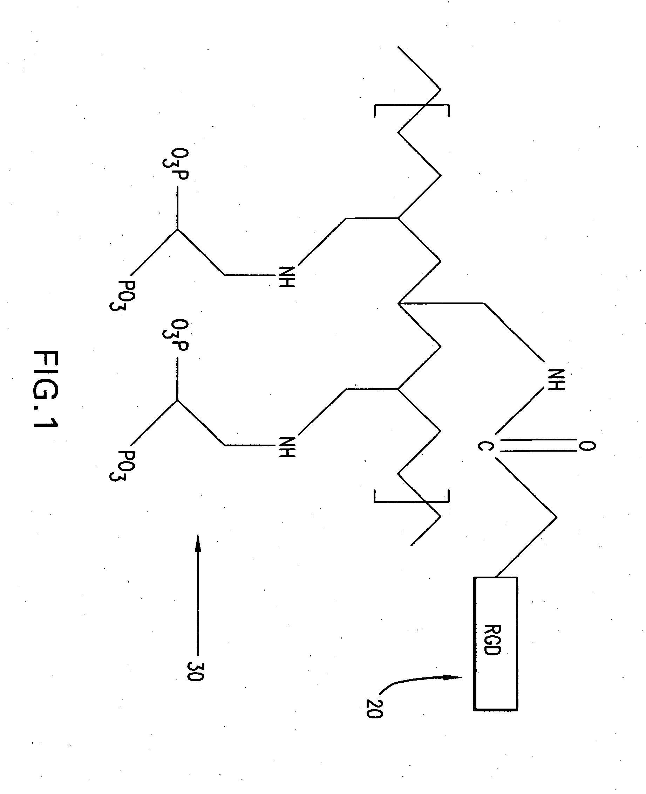

In another example, as shown in FIG. 1, the coating on a medical device may comprise cell adhesion polypeptides 20 (containing RGD in this particular example) grafted onto polybisphosphonate 30. Polybisphosphonates that can be used to coat metallic substrates are described in Fishbein et al., Bisphosphonate-Mediated Gene Vector Delivery From the Metal Surfaces of Stents, Proc. Natl. Acad. Sci. 103(1):159-164 (2006). Some polybisphosphonates, such as polyallylamine bisphosphonate, have amino functional groups which can be coupled to peptides via the carboxy-terminus using a carbodiimide coupling reaction. The polypeptide-grafted polybisphophonate may be coated onto the medical device as a monolayer, such as a Langmuir monolayer or a self-assembling monolayer. As used herein, a "self-assembled monolayer" refers to a relatively ordered assembly of molecules spontaneously chemisorbed on a surface, in which the molecules are oriented approximately parallel to each other and roughly perpendicular to the surface. Each of the molecules includes a functional group that adheres to the surface, and a portion that interacts with neighboring molecules in the monolayer to form the relatively ordered array.

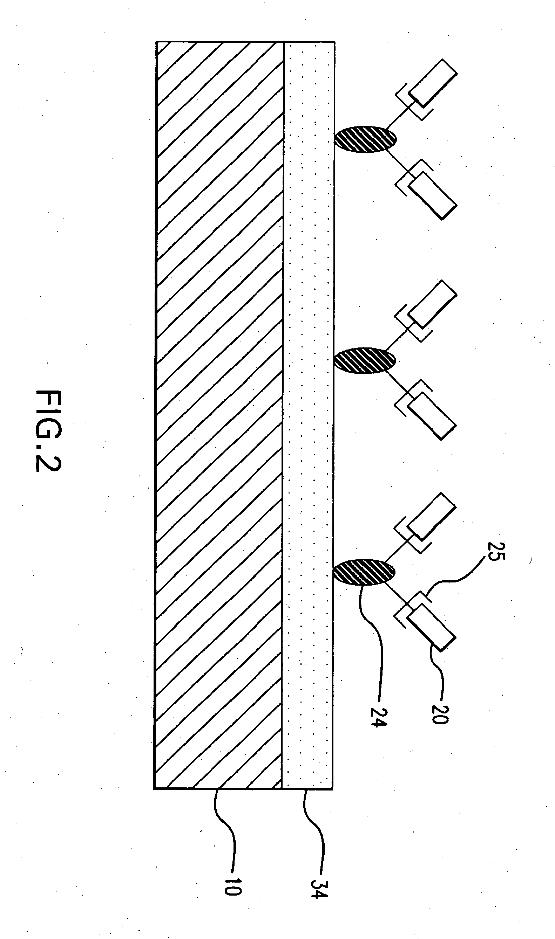

In certain embodiments, the coating on a medical device comprises cell adhesion polypeptides carried on antibodies. As used herein, the term "antibody" refers to an immunoglobulin, whether produced naturally or synthetically (e.g. recombinant), either in whole or in part. The term antibody also encompasses antibody fragments, which refers to any derivative of an antibody that is less than full length while retaining at least a portion of the full-length antibody's specific binding ability. Examples of antibody fragments include, but are not limited to, Fab, Fab', F(ab)₂, F(ab')₂, and Fv. As shown in FIG. 2, the antibodies 24 are conjugated to cell adhesion polypeptides 20 via the antigen binding site 25 of antibodies 24. The cell adhesion polypeptides may be conjugated to the antibodies prior to deployment of the medical device (e.g., during the manufacture of the medical device). Alternatively, it is also possible for the cell adhesion polypeptides to be conjugated to the antibodies after deployment of the medical device (e.g., by intravascular catheter delivery).

Antibodies 24 may be affixed onto a medical device 10 using various methods known in the art, including the method used to make the antibody-coated stents described in U.S. Patent Publication No. 2005/0043787 (Kutryk et al.). For example, medical device 10 may be coated with an antibody binding matrix 34 formed of synthetic materials (e.g., polyurethane, segmented polyurethane-urea/heparin, polylactic acid, cellulose ester, or polyethylene glycol) or naturally occurring materials (e.g., collagen, laminin, heparin, fibrin, cellulose, or carbon). Antibodies 24 are tethered onto the matrix by either covalent or non-covalent bonding.

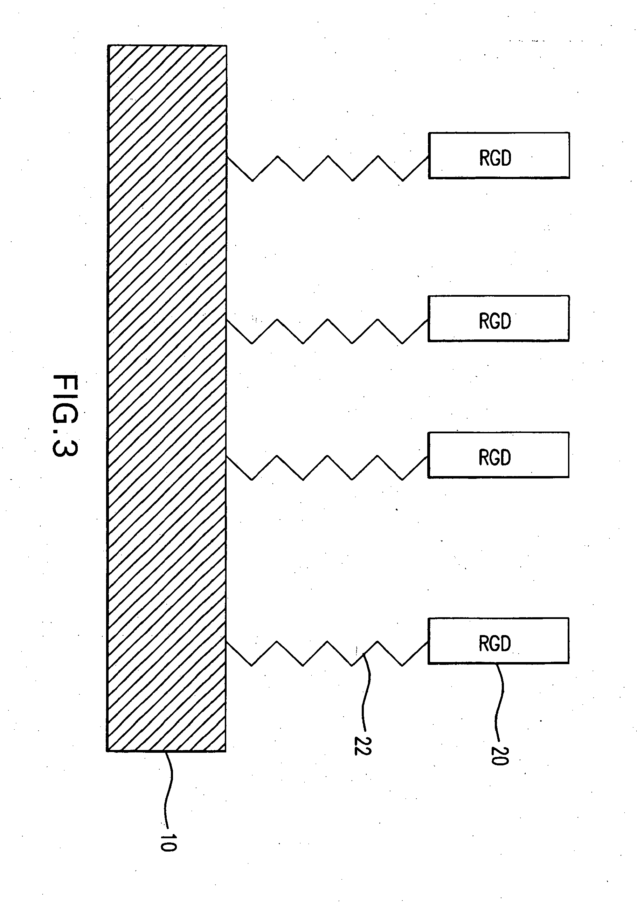

In certain embodiments, the cell adhesion polypeptides may be modified to enhance their adhesiveness to the surfaces of the medical devices. Peptides containing certain amino acids are known to have greater adhesion to inorganic surfaces, as described in Willet et al., Differential Adhesion of Amino Acids to Inorganic Surfaces, Proc. Natal. Acad. Sci. 102(22):7817-7822 (2005). The cell adhesion polypeptides used in the present invention may be modified to include such amino acids to promote adhesion to the surfaces of medical devices. For example, as shown in FIG. 3, a cell adhesion polypeptide 20 may be linked with an adhesive polypeptide segment 22 comprising a sequence of hydrophobic or charged amino acids, such as a polylysine tail. Adhesive polypeptide segment 22 is oriented towards the surface of medical device 10 so as to promote adhesion of polypeptide 20 onto medical device 10. The modified polypeptides may be coated onto the medical device as a monolayer, such as a Langmuir monolayer or a self assembling monolayer.

In certain embodiments, the polypeptides may be displayed on a bacteriophage (phage). Phage display is the expression of polypeptides on the surface of bacteriophage particles. Phage display technology can be used to create phages for displaying a wide variety of polypeptides. *See* Willats, Phage Display: Practicalities and Prospects, Plant Molecular Bio. 50:837-854 (2002).

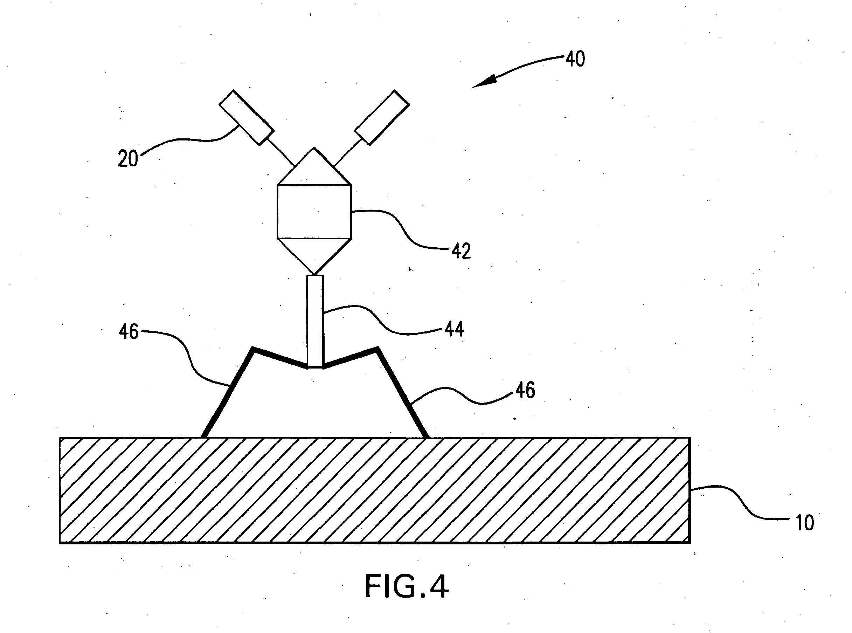

In this embodiment, as shown in FIG. 4, a bacteriophage 40 disposed on the surface of a medical device 10 has a head section 42, a tail section 44, and tail fibers 46. Head section 42 is modified to display polypeptides 20 on its surface. Further, tail fibers 46 are modified to include amino acids (e.g., positively charged amino acids) that would promote adherence to the surface of the medical device. For example, tail fibers 46 may be modified to include a polylysine sequence. Such modifications to the bacteriophage can be made through any conventional genetic engineering process, such as processes for altering the bacteriophage genes encoding for the proteins expressed in the head section and tail fibers.

Because endothelial cells must first migrate onto the stent surface before adhering to the coating of cell adhesion polypeptides, there is an interim period after implantation in which the cell adhesion polypeptide coating on the medical device is vulnerable to biofouling. Thus, in certain embodiments, the cell adhesion polypeptides are provided with a barrier means for temporarily protecting the polypeptides from biofouling. As used herein, the term "biofouling" refers to the binding of non-targeted materials, such as plasma proteins, platelets, and red blood cells, onto the polypeptides or the coating of polypeptides such that it interferes with the binding of targeted endothelial cells.

The temporary barrier is formed of a biodegradable material such as a biodegradable polymer and is designed to degrade upon implantation of the medical device and thereby expose the cell adhesion polypeptides to the physiologic environment in a timeframe coincident with the process of re-endothelialization. For a vascular stent, the process of re-endothelialization is known to begin very shortly after implantation. Time course analysis in rabbits has demonstrated almost 20% stent endothelialization 4 days after implantation and almost 40% after 7 *days. See* Belle et al., Stent Endothelialization: Time Course, Impact of Local Catheter Delivery, Feasibility of Recombinant Protein Administration, and Response to Cytokine Expedition, Circulation 95:438-448 (1997). As well known in the art, the biodegradation rate of a biodegradable polymeric barrier may be controlled by various factors such as the composition, structure, and thickness of the barrier. Therefore, one of ordinary skill in the art can design a biodegradable barrier to degrade and expose the underlying cell adhesion polypeptides in a timeframe coincident with the process of re-endothelialization, while minimizing the opportunity for biofouling of the polypeptides. For example, the biodegradable barrier may be designed to degrade such that the polypeptides are exposed within 4 days or within 7 days after implantation. Certain polymers of poly(L-lactide-co-glycolide) and poly(L-lactide) having various degradation rates are reported in Zilberman et al., Dexamethasone loaded bioresorbable films used in medical support devices: Structure, degradation, crystallinity and drug release, Acta Biomaterialia 1:615-624 (2005). This polymer degradation rate has been shown to be adjustable by varying the lactide to glycolide ratio as well as by varying the chiral configuration of the lactide monomer. In addition, the thickness of the coating can be adjusted to fine tune the rate at which the polypeptides are exposed, yielding a wide range of possible exposure profiles.

Within certain embodiments, the temporary barrier is a biodegradable polymer coating at least partially disposed over the cell adhesion polypeptides. The polypeptides may be disposed on the surface of the medical device by using any method known in the art, including conventional coating techniques such as spray coating, electrostatic spray coating, and dip coating. The polypeptides may also be disposed on the surface of the medical device by any of the methods disclosed in the aforementioned embodiments. The biodegradable coating may be applied onto the medical device using any known method of applying such coatings to the surfaces of medical devices.

Examples of suitable biodegradable polymers include polycarboxylic acid, polyanhydrides including maleic anhydride polymers; polyorthoesters; poly-amino acids; polyethylene oxide; polyphosphazenes; polylactic acid, polyglycolic acid and copolymers and mixtures thereof such as poly(L-lactic acid) (PLLA), poly(D,L-lactide), poly(lactic acid-co-glycolic acid), 50/50 (DL-lactide-co-glycolide); polydioxanone; polypropylene fumarate; polydepsipeptides; polycaprolactone and co-polymers and mixtures thereof such as poly(D,L-lactide-co-caprolactone) and polycaprolactone co-butylacrylate; polyhydroxybutyrate valerate and blends; polycarbonates such as tyrosine-derived polycarbonates and arylates, polyiminocarbonates, and polydimethyltrimethylcarbonates; cyanoacrylate; calcium phosphates; polyglycosaminoglycans; macromolecules such as polysaccharides (including hyaluronic acid; cellulose, and hydroxypropylmethyl cellulose; gelatin; starches; dextrans; alginates and derivatives thereof), proteins and polypeptides; and mixtures and copolymers of any of the foregoing. The biodegradable polymer may also be a surface erodable polymer such as polyhydroxybutyrate and its copolymers, polycaprolactone, polyanhydrides (both crystalline and amorphous), maleic anhydride copolymers, and zinc-calcium phosphate.

In certain embodiments, the temporary barrier comprises a plurality of biodegradable vesicles, wherein the cell adhesion polypeptides are encapsulated within the vesicles. The vesicles may be micelles, liposomes, lipospheres, microspheres, microbubbles, and the like, and may be formed of polymers or lipids. As described above, the vesicle walls can be designed to degrade in a time frame coincident with the process of re-endothelialization. Degradation of the vesicles will release the polypeptides, which can then precipitate onto the medical device surface and adhere thereto. The vesicles may be disposed on the medical device in various ways known in the art. For example, the vesicles may be embedded within a porous surface on the medical device.

Within certain embodiments, the medical device may further comprise therapeutic agents. The therapeutic agent may be carried on or within any component of the medical device, including on or within the temporary protective barrier or another polymer coating on the medical device. In some instances, the therapeutic agent may be provided on a surface of the medical device by any of the methods by which the cell adhesion polypeptides are adhered thereto. In fact, the therapeutic agent may be provided on the same surface as the cell adhesion polypeptides.

The therapeutic agents may be agents that promote angiogenesis or the activation, recruitment, or migration of endothelial cells. For example, angiogenic factors such as PD-ECGF (platelet-derived endothelial cell growth factor) or VEGF (vascular endothelial growth factor), or endothelial cell chemoattractants such as 2-deoxy-D-ribose could be released from the medical device to recruit endothelial cells onto the medical device.

The therapeutic agent may also be any pharmaceutically acceptable agent such as a non-genetic therapeutic agent, a biomolecule, a small molecule, or cells.

Exemplary non-genetic therapeutic agents include anti-thrombogenic agents such heparin, heparin derivatives, prostaglandin (including micellar prostaglandin E1), urokinase, and PPack (dextrophenylalanine proline arginine chloromethylketone); anti-proliferative agents such as enoxaparin, angiopeptin, sirolimus (rapamycin), tacrolimus, everolimus, zotarolimus, monoclonal antibodies capable of blocking smooth muscle cell proliferation, hirudin, and acetylsalicylic acid; anti-inflammatory agents such as dexamethasone, rosiglitazone, prednisolone, corticosterone, budesonide, estrogen, estrodiol, sulfasalazine, acetylsalicylic acid, mycophenolic acid, and mesalamine; anti-neoplastic/anti-proliferative/anti-mitotic agents such as paclitaxel, epothilone, cladribine, 5-fluorouracil, methotrexate, doxorubicin, daunonibicin, cyclosporine, cisplatin, vinblastine, vincristine, epothilones, endostatin, trapidil, halofuginone, and angiostatin; anti-cancer agents such as antisense inhibitors of c-myc oncogene; antimicrobial agents such as triclosan, cephalosporins, aminoglycosides, nitrofurantoin, silver ions, compounds, or salts; biofilm synthesis inhibitors such as non-steroidal anti-inflammatory agents and chelating agents such as ethylenediaminetetraacetic acid, O,O'-bis (2-aminoethyl) ethylcneglycol-N,N,N',N'-tetraacetic acid and mixtures thereof; antibiotics such as gentamycin, rifampin, minocyclin, and ciprofloxacin; antibodies including chimeric antibodies and antibody fragments; anesthetic agents such as lidocaine, bupivacaine, and ropivacaine; nitric oxide;,nitric oxide (NO) donors such as linsidomine, molsidomine, L-arginine, NO-carbohydrate adducts, polymeric or oligoineric NO adducts; anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, enoxaparin, hirudin, warfarin sodium, Dicumarol, aspirin, prostaglandin inhibitors, platelet aggregation inhibitors such as cilostazol and tick antiplatelet factors; vascular cell growth promotors such as growth factors, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; agents which interfere with endogenous vascoactive mechanisms; inhibitors of heat shock proteins such as geldanamycin; angiotensin converting enzyme (ACE) inhibitors; beta-blockers; βAR kinase (βARK) inhibitors; phospholamban inhibitors; protein-bound particle drugs such as ABRAXANE™; and any combinations and prodrugs of the above.

Exemplary biomolecules include peptides, polypeptides and proteins; oligonucleotides; nucleic acids such as double or single stranded DNA (including naked and cDNA), RNA, antisense nucleic acids such as antisense DNA and RNA, small interfering RNA (siRNA), and ribozymes; genes; carbohydrates; angiogenic factors including growth factors; cell cycle inhibitors; and anti-restenosis agents. Nucleic acids may be incorporated into delivery systems such as, for example, vectors (including viral vectors), plasmids or liposomes.

Non-limiting examples of proteins include serca-2 protein, monocyte chemoattractant proteins (MCP-1) and bone morphogenic proteins ("BMP's"), such as, for example, BMP-2, BMP-3, BMP-4, BMP-5, BMP-6 (VGR-1), BMP-7 (OP-1), BMP-8, BMP-9, BMP-10, BMP-11, BMP-12, BMP-13, BMP-14, BMP-15. Preferred BMP's are any of BMP-2, BMP-3, BMP-4, BMP-5, BMP-6, and BMP-7. These BMPs can be provided as homodimers, heterodimers, or combinations thereof, alone or together with other molecules. Alternatively, or in addition, molecules capable of inducing an upstream or downstream effect of a BMP can be provided. Such molecules include any of the "hedghog" proteins, or the DNA's encoding them. Non-limiting examples of genes include survival genes that protect against cell death, such as anti-apoptotic Bcl-2 family factors and Akt kinase; serca 2 gene; and combinations thereof. Non-limiting examples of angiogenic factors include acidic and basic fibroblast growth factors, vascular endothelial growth factor, epidermal growth factor, transforming growth factors α and β, platetet-derived endothelial growth factor, platelet-derived growth factor, tumor necrosis factor α, hepatocyte growth factor, and insulin-like growth factor. A non-limiting example of a cell cycle inhibitor is a cathespin D (CD) inhibitor. Non-limiting examples of anti-restenosis agents include p15, p16, p 18, p 19, p21, p27, p53, p57, Rb, nFkB and E2F decoys, thymidine kinase and combinations thereof and other agents useful for interfering with cell proliferation.

Exemplary small molecules include hormones, nucleotides, amino acids, sugars, and lipids and compounds have a molecular weight of less than 100kD.

Exemplary cells include stem cells, progenitor cells, endothelial cells, adult cardiomyocytes, and smooth muscle cells. Cells can be of human origin (autologous or allogenic) or from an animal source (xenogenic), or genetically engineered. Non-limiting examples of cells include side population (SP) cells, lineage negative (Lin-) cells including LinCD34-, Lin⁻CD34⁺, Lin-cKit ⁺, mesenchymal stem cells including mesenchymal stem cells with 5-aza, cord blood cells, cardiac or other tissue derived stem cells, whole bone marrow, bone marrow mononuclear cells, endothelial progenitor cells, skeletal myoblasts or satellite cells, muscle derived cells, go cells, endothelial cells, adult cardiomyocytes, fibroblasts, smooth muscle cells, adult cardiac fibroblasts + 5-aza, genetically modified cells, tissue engineered grafts, MyoD scar fibroblasts, pacing cells, embryonic stem cell clones, embryonic stem cells, fetal or neonatal cells, immunologically masked cells, and teratoma derived cells. Any of the therapeutic agents may be combined to the extent such combination is biologically compatible.

Non-limiting examples of medical devices that can be used with the present invention include stents, stent grafts, catheters, guide wires, neurovascular aneurysm coils, balloons, filters (*e.g*., vena cava filters), vascular grafts, intraluminal paving systems, pacemakers, electrodes, leads, defibrillators, joint and bone implants, spinal implants, access ports, intra-aortic balloon pumps, heart valves, sutures, artificial hearts, neurological stimulators, cochlear implants, retinal implants, and other devices that can be used in connection with therapeutic coatings. Such medical devices are implanted or otherwise used in body structures, cavities, or lumens such as the vasculature, gastrointestinal tract, abdomen, peritoneum, airways, esophagus, trachea, colon, rectum, biliary tract, urinary tract, prostate, brain, spine, lung, liver, heart, skeletal muscle, kidney, bladder, intestines, stomach, pancreas, ovary, uterus, cartilage, eye, bone, joints, and the like.

## Claims

1. A medical device having a coating er at least a portion of the medical device, wherein the coating comprises:
(a) one or more cell adhesion olypeptides; and
(b) a biodegradable barrier comprising a biodegradable polymer at least partially disposed over or surrounding the one or more cell adhesion polypeptides, wherein the biodegradable barrier substantially degrades within 7 days after implantation of the medical device in a patient.

2. The medical device of claim 1, wherein the cell adhesion polypeptides are peptides derived from a binding domain of a cell adhesion protein of the extracellular matrix.

3. The medical device of claim 2, wherein the cell adhesion protein is selected from the group consisting of fibronectin, vitronectin, laminin, elastin, fibrinogen, collagen type I, collagen type II, and collagen type V.

4. The medical device of claim 2, wherein the peptides comprise an amino acid sequence selected from the group consisting of arginine-glycine-aspartate (RGD) and tyrosine-isoleucine-glycine-serine-arginine (YIGSR).

5. The medical device of claim 1, wherein the cell adhesion polypeptides form a monolayer on the surface of the medical device.

6. The medical device of claim 1, wherein the biodegradable barrier comprises biodegradable micelles, and wherein the polypeptides are encapsulated within the micelles.

7. The medical device of claim 1, wherein the biodegradable barrier substantially degrades within 4 days after implantation of the medical device in a patient.

8. The medical device of claim 1, wherein the cell adhesion polypeptides are grafted onto a polybisphosphonate.

9. The medical device of claim 1, wherein the cell adhesion polypeptides are linked to adhesive polypeptide segments.

## Patentansprüche

1. Eine medizinische Vorrichtung, welche mindestens auf einem Teil der medizinischen Vorrichtung eine Beschichtung aufweist, wobei die Beschichtung umfasst:
(a) ein oder mehrere Zelladhäsionspolypeptide; und
(b) eine ein bioabbaubares Polymer umfassende bioabbaubare Barriere, welche mindestens teilweise auf dem einen oder mehreren Zelladhäsionspolypeptiden platziert ist oder diese umgibt, wobei die bioabbaubare Barriere innerhalb von sieben Tagen nach Implantation der medizinischen Vorrichtung in einen Patienten im Wesentlichen zerfällt.

2. Die medizinische Vorrichtung nach Anspruch 1, wobei die Zelladhäsionspolypeptide Peptide sind, welche sich von einer Bindungsdomäne eines Zelladhäsionsproteins der extrazellulären Matrix ableiten.

3. Die medizinische Vorrichtung nach Anspruch 2, wobei das Zelladhäsionsprotein aus der aus Fibronektin, Vitronektin, Laminin, Elastin, Fibrinogen, Kollagen Typ I, Kollagen Typ II und Kollagen Typ V bestehenden Gruppe ausgewählt ist.

4. Die medizinische Vorrichtung nach Anspruch 2, wobei die Peptide eine Aminosäuresequenz umfassen, welche aus der aus Arginin-Glycin-Aspartat (RGD) und Tyrosin-Isoleucin-Glycin-Serin-Arginin (YIGSR) bestehenden Gruppe ausgewählt ist.

5. Die medizinische Vorrichtung nach Anspruch 1, wobei die Zelladhäsionspolypeptide eine Monoschicht auf der Oberfläche der medizinischen Vorrichtung bilden.

6. Die medizinische Vorrichtung nach Anspruch 1, wobei die bioabbaubare Barriere bioabbaubare Mizellen umfasst, und wobei die Polypeptide innerhalb der Mizellen eingekapselt sind.

7. Die medizinische Vorrichtung nach Anspruch 1, wobei die bioabbaubare Barriere innerhalb von vier Tagen nach Implantation der medizinischen Vorrichtung in einen Patienten im Wesentlichen zerfällt.

8. Die medizinische Vorrichtung nach Anspruch 1, wobei die Zelladhäsionspolypeptide auf ein Polybisphosphonat gepfropft sind.

9. Die medizinische Vorrichtung nach Anspruch 1, wobei die Zelladhäsionspolypeptide mit adhäsiven Polypeptidsegmenten verbunden sind.

## Revendications

1. Dispositif médical ayant un revêtement sur au moins une partie du dispositif médical, où le revêtement comprend :
(a) un ou plusieurs polypeptides d'adhésion cellulaire ; et
(b) une barrière biodégradable comprenant un polymère biodégradable disposée au moins partiellement sur ou entourant le un ou plusieurs polypeptides d'adhésion cellulaire, où la barrière biodégradable se dégrade sensiblement dans un intervalle de 7 jours après l'implantation du dispositif médical dans un patient.

2. Dispositif médical selon la revendication 1, où les polypeptides d'adhésion cellulaire sont des peptides dérivés d'un domaine de liaison d'une protéine d'adhésion cellulaire de la matrice extracellulaire.

3. Dispositif médical selon la revendication 2, où la protéine d'adhésion cellulaire est choisie dans le groupe consistant en fibronectine, vitronectine, laminine, élastine, fibrinogène, collagène type I, collagène type II, et collagène type V.

4. Dispositif médical selon la revendication 2, où les peptides comprennent une séquence d'aminoacide choisie dans le groupe consistant en arginine-glycine-aspartate (RGD) et tyrosine-isoleucine-sérine-arginine (YIGSR).

5. Dispositif médical selon la revendication 1, où les polypeptides d'adhésion cellulaires forment une monocouche sur la surface du dispositif médical.

6. Dispositif médical selon la revendication 1, où la barrière biodégradable comprend des micelles biodégradables, et où les peptides sont encapsulés dans les micelles.

7. Dispositif médical selon la revendication 1, où la barrière biodégradable se dégrade sensiblement dans un intervalle de 4 jours après l'implantation du dispositif médical dans un patient.

8. Dispositif médical selon la revendication 1, où les peptides d'adhésion cellulaire sont greffés sur un polybisphosphonate.

9. Dispositif médical selon la revendication 1, où les peptides d'adhésion cellulaire sont liés à des segments de polypeptides adhésifs.


### SEQUENCE LISTING

<110> BENCO, JOHN BODEN, MARK BRITO, SHAINA NAIMARK, WENDY PHAM, LAN
<120> MEDICAL DEVICES HAVING A COATING FOR PROMOTING ENDOTHELIAL CELL ADHESION
<130> R1362 EP
<140> EP 07 83 7562.3
   <141> 2007-08-30
<150> 60/842,384
   <151> 2006-09-06
<160> 2
<170> PatentIn ver. 3.3
<210> 1
   <211> 3
   <212> PRT
   <213> Artificial Sequence
<220>
   <223> /note="Description of Artificial sequence: Synthetic peptide"
<400> 1
   Arg Gly Asp
   1
<210> 2
   <211> 5
   <212> PRT
   <213> Artificial sequence
<220>
   <223> /note="Description of Artificial sequence: synthetic peptide"
<400> 2
   Tyr Ile Gly Ser Arg
   1               5